# EUROPEAN PATENT APPLICATION

(11) **EP 4 582 458 A1**
(43) Date of publication of application: **09.07.2025**
(21) Application number: 23860829.3
(22) Date of filing: 29.08.2023
(51) Int. Cl.: C07K 19/00, C07K 7/06, A61K 39/385, A61K 39/00

(54) **MICELLE COMPRISING AMPHIPHILIC PEPTIDE, AND ANTIGEN CARRIER NANOPARTICLE USING SAME**

(30) Priority: 29.08.2022 KR 20220108301
(71) Applicant: RTAB Co., Ltd., Seoul 04385 (KR)
(72) Inventor: RHIM, Taiyoun, Seoul 06709 (KR); LEE, Minhyung, Gwacheon-si, Gyeonggi-do 13836 (KR); KIM, Sung Il, Seoul 04763 (KR)
(74) Representative: Dehns
(86) International application number: PCT/KR2023/012758
(87) International publication number: WO 2024/049153

(57) **Abstract**

The present invention relates to a nanoparticle and a preparation method therefor, the nanoparticle comprising an amphiphilic peptide, which forms a micelle structure through self-assembly, and a target peptide (preferably, a water-soluble antigen peptide), which electrically binds to the surface of the amphiphilic peptide. The target peptide electrically binds to the surface of the amphiphilic peptide micelle structure and becomes particulated, and thus can be effectively presented to an antigen-presenting cell, and the weight ratio of the amphiphilic peptide and the target peptide is controlled so that the size of nanoparticles is controlled and endocytosis thereof is carried out, and thus immunity by means of cytotoxic T cells can be induced. Nanoparticles of the present invention exhibit use only an epitope of a more accurate region so as to be effective as a vaccine, and thus have minimal side effects. Therefore, the present invention exhibits excellent antigen-specific antibody and cell immunotherapy effects, and thus can be used in various fields such as vaccine production.

## Description

### Technical Field

The present invention provides a micelle structure which is composed of an amphiphilic peptide, and provides a nanoparticle in which a target peptide is bound to a surface of the micelle structure. According to the present invention, the nanoparticles are used as antigen carriers and have an effect as a vaccine.

The present invention was developed with support from the following national research and development project.
[Project Unique Number] 1711163105
[Project Number] 2021R1A2C1013022
[Name of Ministry] Ministry of Science and ICT
[Name of Project Management (Specialized) Institution] National Research Foundation of Korea
[Title of Research Project] Individual Basic Research (Ministry of Science and ICT)
[Title of Research Task] Particle antigenization of water-soluble incomplete antigen using amphiphilic peptides
[Name of Task Performance Institution] Hanyang University

### Background Art

The process of forming antibodies, which play an important role in our body's immune function, can be broadly divided into two types depending on the type of antigen. Particulate antigens are recognized by pattern recognition receptors that are present in macrophages and dendritic cells, are incorporated into cells through phagocytosis, and are degraded by the proteasome, and afterwards, they are presented on MHC II molecules. On the other hand, soluble antigens are recognized by cells with receptors therefor, enter the cells through receptor-mediated endocytosis, and then are degraded and presented on MHC II molecules.

In general, it is known that antibodies against particulate antigens are easily produced. Dendritic cells and macrophages, which are evenly distributed in each tissue for defense, react non-specifically to particulate antigens and produce antibodies against the same. On the other hand, soluble antigens are recognized mainly by B cells and cells differentiated from B cells that have specific receptors therefor. However, since B cells are not evenly distributed in all tissues and are mainly located inside the lymph nodes, a migration process must be accompanied (Curr Opin Immunol, 65, 1-6, 2020), and there must be a receptor capable of binding to the antigen. In addition, since B cells have B cell receptors with different characteristics for each cell, not only the production of antibodies to soluble antigens, but also the persistence of antibodies produced is significantly lower than that of particle antigens.

Meanwhile, when antibodies are produced in the form of haptens using some amino acid sequences or when the regions exposed outside the cell are made into recombinant proteins using methods such as gene truncation and used as antigens, accessibility problems arise for antibodies, and in addition to the problem of creating a chemical bond (covalent bond) between the hapten and the carrier protein, structural changes can occur, and side effects can occur as antibodies to this part are created or antibodies to the large carrier protein are created (Sci. Adv. 6, eaaax2285, 2020). In order to solve the problem that water-soluble antigens have a significantly lower antibody production rate *in vivo* compared to particle antigens, attempts have been made to develop various adjuvants such as nanoparticles, but due to low efficacy and stability, unacceptable tolerability, difficulty in manufacturing, toxicity and the like, no successful alternative has been developed as of yet (Hum. Vacc. Imm, 10: 9, 2761-2774, 2014).

When producing vaccines against nanoparticles such as bacteria or fungi, the ability to produce antibodies is usually not a major consideration. Traditional vaccines in which pathogens are weakened (attenuated vaccines) or inactivated (inactivated vaccines) have been widely used due to their high antibody production capacity. In these cases, there were issues with the toxicity and safety of the vaccine itself rather than its ability to produce antibodies.

In the case of vaccines against viruses, there are many viruses that cannot be attenuated or inactivated, and in vaccines against cancer, attenuation or inactivation is impossible because both cancer cell-specific and non-specific antigens exist together. In order to solve this problem, subunit vaccines and recombinant vaccines have been developed as second-generation vaccines, but there is a problem in that antibodies cannot be formed effectively due to the limitations of water-soluble antigens as described above.

Meanwhile, the basic concept of the third-generation vaccines is that if the delivery chain platform is secured, once the target is determined, a vaccine can be used by making a module of DNA or RNA that can produce (encode) the target molecule, and thus, it was possible to develop a new vaccine in a very short period of time. The reality is that many researchers are developing safer and more stable carrier platforms.

However, mRNA vaccines must make proteins in the body, and thus cannot be made smaller than a certain length (if they are too small, the level of protein expression decreases). Therefore, there is a limitation in that it is difficult to perform precise targeting.

An example of vaccine development that requires sophisticated targeting is a dengue fever vaccine. As the number of patients with dengue fever virus continues to increase and the number of critically ill patients also increases, the need to develop a vaccine therefor has also increased, and several research entities are working on development. The first licensed product was released in 2018, but as a result of clinical use, significant side effects were observed, and thus, its use was restricted.

Currently, four types of viruses that cause dengue fever are known. Antibodies are mainly formed against the surface antigen, E protein, and the amino acid sequence of this E protein appears very similar in the four types of viruses. More than 52% of the amino acid sequences are completely identical, and an overall similarity of more than 80% is observed. In other words, the sequences of the E protein, which is a surface protein of the four types of dengue viruses, are more than 80% similar. The biggest problem with the dengue fever vaccine is that it has a preventive effect if the correct antibody is produced and neutralizing antibodies are produced, but if it does not bind accurately as a divalent but rather binds weakly to a similar antigen, the problem of increased infection may be caused by antibody-dependent enhancement.

Therefore, in order to produce a multivalent vaccine with minimal side effects, it is necessary to construct a vaccine with only antigenic determinants from more precise regions. However, existing nucleic acid vaccine platforms cannot meet these needs. Accordingly, the inventors of the present invention completed the present invention in order to provide an antigen that targets only the modified portion of the virus or tumor marker.

### Disclosure

### Technical Problem

One aspect is to provide a nanoparticle including an amphiphilic peptide that forms a micelle structure by self-assembly and a target peptide (e.g., a water-soluble antigen peptide) that electrically binds to a surface of the amphiphilic peptide.

Another aspect is to provide a method for preparing a nanoparticle, including forming a micelle structure by mixing an amphiphilic peptide in an aqueous solution; and mixing a target peptide (e.g., water-soluble antigen peptide) to electrically bind to a surface of the formed micelle structure.

Still another aspect of the present invention is to provide a nanoparticle for a multivalent vaccine with minimized side effects by using only epitopes of more precise regions.

### Technical Solution

In order to improve the limitations of the related art, the inventors of the present invention have developed a method in which various amphiphilic peptides having small molecular weights of about 10 amino acids or less can form self-particulated micelles and electrically bind haptenic peptides to a micelle surface thereof to present the same to antigen-presenting cells.

Hereinafter, the present invention will be described in detail.

The terms used in the present application are merely used to describe specific embodiments and are not intended to restrict the invention. Unless otherwise defined, all terms used herein, including technical or scientific terms, have the same meaning as commonly understood by a person of ordinary skill in the art to which the present invention pertains.

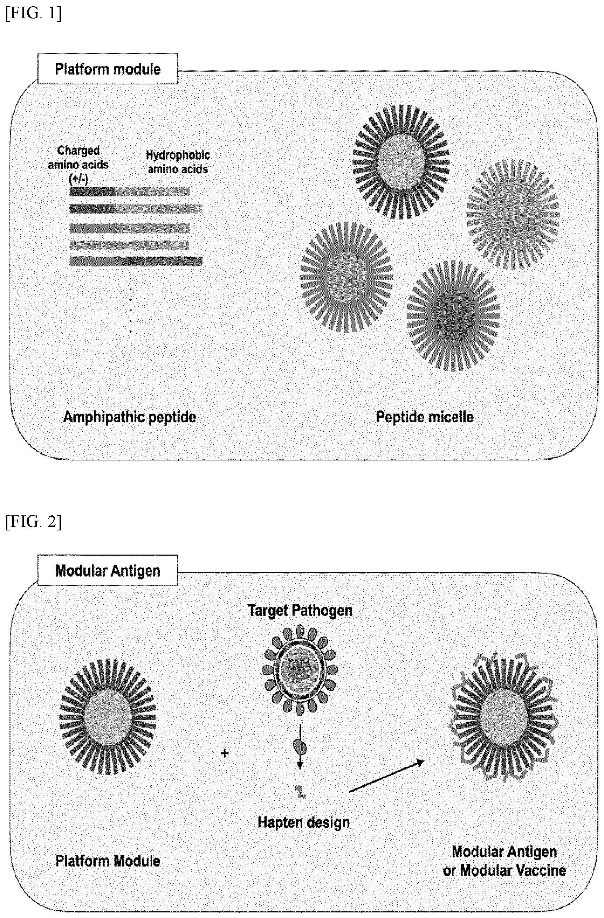

The present invention provides a nanoparticle, including an amphiphilic peptide that forms a micelle structure by self-assembly (see FIG. 1) and a target peptide that electrically binds to a surface of the amphiphilic peptide (or a micelle structure composed of an amphiphilic peptide).

As used herein, the term "amphiphilic peptide" refers to a peptide including both a polar hydrophilic amino acid and a non-polar hydrophobic amino acid. Amphiphilic peptides have the above amino acid constitution and can interact with both hydrophilic and hydrophobic molecules.

In one embodiment of the present invention, the amphiphilic peptide may include 1 to 10 amino acids (or 1 to 15 amino acids), and in one embodiment of the present invention, the amphiphilic peptide is a peptide represented by General Formula I below:

[General formula I] CnXm

wherein C is a charged amino acid, X is a hydrophobic amino acid, n is an integer from 1 to 5, and m is an integer from 3 to 10.

That is, the nanoparticle of the present invention may include a micelle formed of an amphiphilic peptide represented by General Formula I: CnXm (I) (wherein C is a charged amino acid, X is a hydrophobic amino acid which is the same or different amino acid, n is an integer from 1 to 5, and m is an integer from 3 to 10). In one embodiment, the number of charged amino acids may be 2 to 5, and the number of hydrophobic amino acids may be 4 to 8.

In one embodiment of the present invention, the amphiphilic peptide may include an amino acid such as arginine (R), histidine (H) and lysine (K) as a hydrophilic amino acid, which has a positive charge, or an amino acid such as aspartic acid (D), glutamic acid (E), which has a negative charge. In one embodiment, the hydrophilic amino acid may be located on a surface of the micelle to cause the surface of the micelle to have a positive or negative charge.

In one embodiment of the present invention, the amphiphilic peptide may include at least one selected from the group consisting of valine, phenylalanine, tryptophane, isoleucine, leucine, proline, methionine and alanine as hydrophobic amino acids.

In one embodiment of the present invention, the amphiphilic peptide may include arginine (R) as a hydrophilic amino acid and valine (V) as a hydrophobic amino acid.

In one embodiment of the present invention, the amphiphilic peptide may be R3V6 (RRRVVVVVV, SEQ ID NO: 1) consisting of an amino acid sequence of SEQ ID NO: 1 including three arginines as positively charged hydrophilic amino acids and six valines as hydrophobic amino acids, R4V7 (RRRRVVVVVVV, SEQ ID NO: 2) consisting of an amino acid sequence of SEQ ID NO: 2 including four arginines as positively charged hydrophilic amino acids and seven valines as hydrophobic amino acids, E3V6 (EEEVVVVVV, SEQ ID NO: 3) consisting of an amino acid sequence of SEQ ID NO: 3 including three glutamic acids as negatively charged hydrophilic amino acids and six valines as hydrophobic amino acids, or E4V7 (EEEEVVVVVVV, SEQ ID NO: 4) consisting of an amino acid sequence of SEQ ID NO: 4 including four glutamic acids as negatively charged hydrophilic amino acids and seven valines as hydrophobic amino acids.

In one embodiment of the present invention, the 'target peptide' is a peptide that (electrically) binds to an amphiphilic peptide. The 'target peptide' of the present invention is preferably an 'antigen peptide', and for example, it may be a 'hapten peptide'.

In the present invention, the term 'hapten peptide' refers to a low-molecular substance (e.g., low-molecular peptide, low-molecular toxin, etc.) that can electrically bind to the surface of a micelle formed of the amphiphilic peptide. Hapten has a small molecular weight and small size such it cannot alone produce antibodies in response to an immune response in the body, but *in vitro,* it can act as a specific antigen that reacts with the corresponding antibody to form a precipitate of antigen-antibody reaction. Additionally, haptens can produce antibodies when combined with a carrier large enough to induce antibodies, such as a carrier protein.

In one embodiment of the present invention, the 'hapten peptide' consists of 6 to 15 amino acids or 8 to 12 amino acids, and preferably has an amino acid sequence of around 10 amino acids.

The 'target peptide' of the present invention as a whole preferably exhibits a 'positive charge' or a 'negative charge', and has a charge opposite to a surface charge of the micelle structure. The target peptide of the present invention has, for example, a charge of +1, +2, +3, -1, -2 or -3.

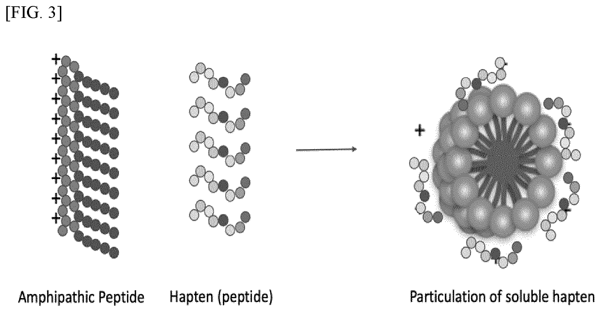

In one embodiment of the present invention, the target peptide may bind to the surface of an amphiphilic peptide. Specifically, the target peptide may be electrically bound to the amphiphilic peptide with opposite polarity (see FIGS. 2 and 3).

In a preferred embodiment of the present invention, the 'hapten peptide' may consist of the amino acid sequences of SEQ ID NO: 5 to 11.

In one embodiment of the present invention, the hapten may be a peptide prepared based on a partial amino acid sequence of the DDOST protein (dolichyl-diphosphooligosaccharide-protein glycosyltransferase 48 kDa subunit precursor glycosyltransferase, NP_005207). For example, the hapten peptide of the present invention may consist of any one of sequences of SEQ ID NO: 5 to 7. SEQ ID NO: 5 (SDLGQHTLIV) of the present invention shows charge -1 in a neutral solution, SEQ ID NO: 6 (LEDTLSSE) shows charge -2 in a neutral solution, and SEQ ID NO: 7 (PGSQRYSQTGN) shows charge + 1 in a neutral solution. However, any 'hapten' that is a compound that can only electrically bind to the surface of a carrier peptide by carrying a charge and forms antibodies against KLH protein or albumin protein when chemically bound to the same and injected into a living body may be used as the hapten peptide proposed in the present invention.

In one embodiment of the present invention, the size of the nanoparticles may be adjusted according to the weight ratio of the amphiphilic peptide serving as a carrier and the target peptide. For example, the size of the nanoparticles may be adjusted by adjusting the weight ratio (w/w) of the amphiphilic peptide and the target peptide to 1:5 to 5:1, 1:4 to 4:1, or 1:3 to 3:1.

In one embodiment of the present invention, the nanoparticle may include an amphiphilic peptide and a hapten at a weight ratio (w/w) of 1:3 to 3:1. Specifically, when the amphiphilic peptide and the hapten peptide were mixed at a weight ratio of 3:1 and 1:3, it was confirmed that as a result of analyzing the particle size using Zeta sizer, the particle size could be controlled to 60 to 150 nm and 300 to 800 nm, respectively. Depending on the size of the nanoparticles, antibody immunity or cellular immunity may be induced.

In one embodiment of the present invention, the size of the nanoparticle may be formed to be 50 nm to 150 nm, 50 nm to 300 nm, 50 nm to 700 nm, 150 nm to 400 nm, or 100 nm to 300 nm, and preferably, it may have a size of 150 nm to 350 nm.

In one embodiment of the present invention, the size of the nanoparticles may be 60 to 150 nm or 300 nm to 800 nm when the amphiphilic peptide and the hapten peptide are included at a weight ratio of 1:3 or 3:1. In this case, the nanoparticles increased phagocytosis by macrophages (see FIG. 1) and were presented on the surface by antigen presenting cells (APC), and thus, it was confirmed that it was possible to increase the production of immunoglobulins more effectively.

In one embodiment of the present invention, the size of the nanoparticles may be 60 to 150 nm and 300 nm to 800 nm when the amphiphilic peptide and the hapten peptide are included at a weight ratio of 3:1 and 1:3. In this case, it was confirmed that the phagocytosis of the nanoparticles into macrophages occurred relatively less (see FIG. 1), and as a result, the formation of immunoglobulins was reduced. On the other hand, it was confirmed that cytotoxic T lymphocytes specific for hapten peptides increased more than helper T cells or B cells specific for hapten peptides.

In addition, the present invention provides a method for preparing a nanoparticle, including forming a micelle structure by mixing an amphiphilic peptide in an aqueous solution; and

mixing a target peptide to electrically bind to a surface of the formed micelle structure.

In one embodiment of the present invention, it may be a method for preparing a nanoparticle, wherein the amphiphilic peptide is a peptide represented by General Formula I below:

[General formula I] CnXm

wherein C is a charged amino acid, X is a hydrophobic amino acid, n is an integer from 1 to 5, and m is an integer from 3 to 10.

In one embodiment of the present invention, the forming a micelle structure may be performed in a glucose solution with a concentration of 5 to 20%. Specifically, by mixing an amphiphilic peptide and a hapten peptide in a glucose solution with a concentration of 5 to 20%, a more stable micelle structure may be formed.

In one embodiment of the present invention, the forming a micelle structure may be performed by further including cholesterol at a weight ratio of 1:5 to 5:1 with respect to the amphiphilic peptide. Specifically, by mixing the amphiphilic peptide and cholesterol at a mass ratio of 1:5, 1:3, 1:1 and the like and then leaving the same at room temperature for 2 hours, particles with relatively small size deviation may be produced.

In one embodiment of the present invention, the method for preparing a nanoparticle may be characterized in that the amphiphilic peptide and the target peptide are mixed at a weight ratio of 1:5 to 5:1 to form particles of the target peptide.

In one embodiment of the present invention, the antigen peptide in the particle generation step may be a hapten peptide.

Additionally, the present invention provides a vaccine composition including the nanoparticle. The vaccine composition may be used in immunotherapy.

The vaccine composition of the present invention may target dengue virus infection, severe fever with thrombocytopenia syndrome or influenza virus infection, but is not limited thereto.

In one embodiment of the present invention, the vaccine may be used to prevent or treat a disease using immune cells (B cells or T cells).

As used herein, the term "prevention" may refer to any act of suppressing or delaying the onset of a disease in a subject by administering a pharmaceutical composition according to one aspect.

As used herein, the term "treatment" may refer to any action in which the symptoms of a disease are ameliorated or beneficially changed by the administration of a pharmaceutical composition according to one aspect.

Additionally, the present invention provides a pharmaceutical composition for preventing or treating an immune disease, including the nanoparticle.

According to one embodiment of the present invention, the immune disease may target any disease caused by pathogenic substances or diseases caused by infection by pathogens such as viruses and microorganisms, and specifically, the pathogen itself or a part thereof or the pathogenic substance may be applied as a hapten. The pharmaceutical composition may further include a pharmaceutically acceptable carrier or diluent. The pharmaceutically acceptable carrier or diluent may be well known in the art. The carrier or the diluent may be lactose, dextrose, sucrose, sorbitol, mannitol, starch, acacia rubber, calcium phosphate, alginate, gelatin, calcium silicate, micro-crystalline cellulose, polyvinyl pyrrolidone, cellulose, water (e.g., saline solution and sterile water), syrup, methyl cellulose, methylhydroxy benzoate, propylhydroxy benzoate, talc, magnesium stearate, mineral oil, Ringer's solution, buffer solution, maltodextrin solution, glycerol, ethanol, dextran, albumin or any combination thereof. The pharmaceutical composition may further include a lubricant, a humectant, a sweetener, a flavoring agent, an emulsifier, a suspension or a preservative.

The pharmaceutical composition may be formulated with a pharmaceutically acceptable carrier and/or excipient into a unit dosage form or a multiple dosage form by a well-known method in the art. In this case, the formulation may be a solution in oil or aqueous medium, a suspension, a syrup or an emulsifying solution, an extract, a powder, a granule, a tablet or a capsule, and may further include a dispersant or a stabilizer. The aqueous medium may include a saline solution or a phosphate buffer solution (PBS). The pharmaceutical composition according to an embodiment may be formulated in the form of an oral or parenteral administration, preferably, in the form of a parenteral administration. In general, a sterile solution of an active ingredient is prepared, and a buffer for adjusting the pH is added to the solution for intramuscular, intraperitoneal, transdermal and intravenous administration. For intravenous administration, an isotonic agent may further be added to a preparation for isotonicity.

The dosage (effective amount) of the pharmaceutical composition according to an embodiment may be prescribed in various ways depending on factors such as the formulation method, administration method, patient's age, weight, gender, pathological condition, food, administration time, administration route, excretion rate and response sensitivity, and a person skilled in the art may appropriately adjust the dosage by considering these factors. The frequency of administration may be once, or twice or more within the range of clinically acceptable side effects, and the site of administration may be one, two or more sites, every day or at every 2 or 5 days for a total duration of 1 day to 30 days for each treatment. If required, the same treatment may be repeated after a predetermined period. For animals other than humans, a dosage that is the same as that of per kg in a human, or a dosage that is determined by, for example, conversion based on the volume ratio (e.g. average value) of organs (e.g. heart) of the target animal and a human, may be administered. Available administration routes may include parenteral administration (e.g., subcutaneous, intramuscular, intra-arterial, intraperitoneal, transdermal or intravenous administration), topical administration (including transdermal administration), and injection, or insertion of or a transplantable device or a substance. As a target animal for treatment according to an embodiment, a human and other mammals of interest may be exemplified, and specifically, it may be a human, a monkey, a mouse, a rat, a rabbit, sheep, a cow, a dog, a horse, a pig or the like.

In addition, the present invention provides a health functional food for preventing or improving an immune disease, including the nanoparticle as an active ingredient.

The nanoparticle, immune disease and prevention are the same as described above.

As used herein, the term "amelioration" may refer to any action that suppresses or delays the onset of an immune disease in a subject by administering a composition according to one aspect.

The health functional food as defined herein may be a health functional food as newly defined in the Act on Health Functional Foods revised in 2008, whose functionality and safety in the human body have been sufficiently established and is included in the regulations on functional raw material qualification for health functional foods prescribed in Korean Food and Drug Administration Notice No. 2008-72.

When the composition of the present invention is used by being included in a health functional food, the composition may be added as it is, or may be used along with other health functional foods or health functional food ingredients, or may be appropriately used according to a usual method. A mixed amount of an active ingredient(s) may be determined to be appropriate for the purpose of use. In general, the amount of the active ingredient constituting the composition according to the present invention may be about 0.01 wt% to about 15 wt%, for example, about 0.2 wt% to about 10 wt% with respect to a total weight of the food. When it is prepared as a beverage, based on 100 mL of the beverage, 0.1 g to 30 g, for example, 0.2 g to 5 g of the active ingredient in the composition may be contained. The beverage may entirely consist of natural ingredients. However, in the case of long-term intake for health control and hygienic purposes, the amount of the active ingredient may be equal to or below the above ranges. Since there are no problems in terms of safety, the active ingredient may be used in an amount equal to or above the ranges.

The composition for a health functional food according to the present invention may be formulated in the form of a typical health functional food known in the art. The health functional food may be manufactured into, for example, powders, granules, tablets, pills, capsules, suspensions, emulsions, syrups, precipitates, liquids, extracts, gums, teas, jellies, beverages or the like, and are preferably formulated in the form of beverages. As the sitologically acceptable carrier or additive, any carrier or additive known in the art to be usable in the manufacture of the dosage form to be manufactured may be used. In addition, it may also include food used as feed for animals.

According to purposes or preferences, the health functional food may contain nutrients, vitamins, electrolytes, flavoring agents, coloring agents, pectic acid and salts thereof, alginic acid and salts thereof, organic acids, protective colloid thickening agents, pH adjusting agents, stabilizers, preservatives, glycerin, alcohols, carbonating agents used in carbonated beverages or the like. Fruit flesh for the preparation of other natural fruit juices, fruit juice beverages and vegetable beverages may be contained. In addition, the composition for a health functional food may further include a food additive. Unless prescribed otherwise, acceptability as a "food additive" may be determined based on the specification of an item of concern and standards therefor according to the general provisions and general test methods prescribed in the Korean Food Additives Codex approved by the Korea Ministry of Food and Drug Safety.

In addition, the present invention provides the use of a nanoparticle in the manufacture of a medicine for preventing or treating an immune disease.

### Advantageous Effects

According to the present invention, when an amphiphilic peptide that forms a micelle structure by self-assembly and a target peptide (e.g., a water-soluble antigen peptide) that electrically binds to a surface of the amphiphilic peptide are mixed at a certain weight ratio, the size of the nanoparticles may be adjusted. Depending on its size, it may be presented to helper T lymphocytes to induce the production of antibodies or activate cytotoxic T lymphocytes and cellular immunity, and thus, it is possible to exhibit a target peptide-specific immunotherapeutic effect.

In the present invention, nanoparticles may generate antibodies *in vivo* by inducing immune responses of antigen-presenting cells, B cells and helper T cells without chemical modification, and may induce the production of target peptide-specific cytotoxic T lymphocytes. Therefore, since it can be utilized in areas such as vaccine manufacturing, as well as cellular immunity that has not been implemented to practical use yet, it can be utilized to prevent and treat various diseases such as various viral diseases, cancer and the like.

The nanoparticle of the present invention may be used as a multivalent vaccine with minimal side effects because it can construct only antigenic determinants at more precise sites than before by using hapten peptides as target peptides. In other words, the nanoparticle (vaccine platform) of the present invention may provide an antigen that targets only the modified part of a virus or tumor marker.

### Description of Drawings

FIG. 1 is a mimetic diagram confirming micelle formation conditions for all possible amphiphilic peptides in order to secure the nanoparticles (vaccine platform) targeted by the present invention. Nanoparticles (peptide platform) of the present invention refer to amphiphilic peptides that can form micelles through self-assembly in an aqueous solution. In addition, when the formed micelles are decomposed within an antigen-presenting cell and presented on MHC class 2 molecules, antibodies can also be generated against the micelle-forming peptide, and thus, in order to suppress the formation of antibodies therefrom as much as possible, the micelle is composed of only two types of amino acids that form bipolarity.
FIG. 2 is a mimetic diagram showing the production steps of nanoparticles (Modular Antigen in FIG. 2). According to the present invention, a target peptide (hapten peptide) binds to the surface of a micelle formed of an amphiphilic peptide to form nanoparticles.
FIG. 3 is a mimetic diagram of the formation of nanoparticles (peptide vaccine platform) according to the present invention. The nanoparticles of the present invention are formed by adding a target peptide (preferably, a hapten peptide) to an amphiphilic peptide and then particulating the same.

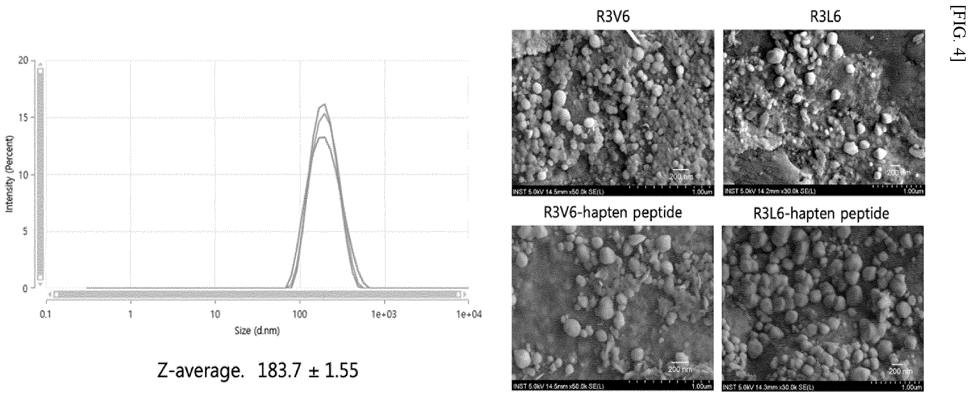

FIG. 4 shows images (left) and an electron microscope image (right) for measuring the size of particles formed by an amphiphilic peptide (R3V6) and an R3V6 (or R3L6)-hapten peptide according to an embodiment of the present invention. As shown in FIG. 4, micelles composed of only amphiphilic peptides form spherical particles with a size between 100 and 300 nm, and when a hapten peptide is electrically attached to an amphiphilic peptide, particles with a size between 150 and 350 nm are formed.

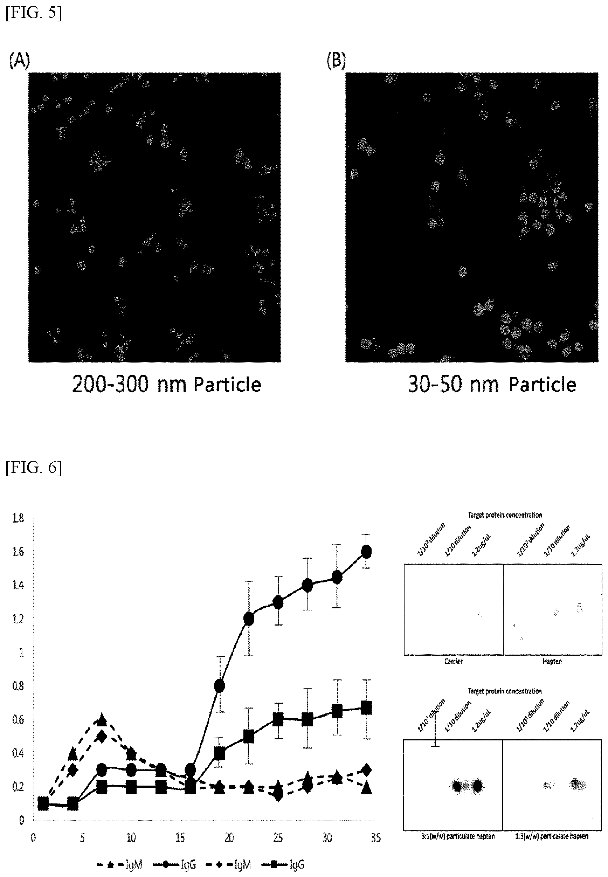

FIG. 5 shows images photographed with a confocal fluorescence microscope of nanoparticles internalized in macrophages according to their size. There is a difference in the degree of uptake by macrophage cell lines depending on the size of the manufactured nanoparticles, and it can be confirmed that modular vaccines with an average size of about 200 to 300 nm were well absorbed.
FIG. 6 is a graph showing the expression levels of target protein-specific IgG and IgM antibodies in mouse blood according to the administration of nanoparticles produced according to the present invention. When nanoparticles with a size of 200 to 300 nm were treated (●), immunogenicity was greater than when nanoparticles with a size of 30 to 50 nm were administered (■).

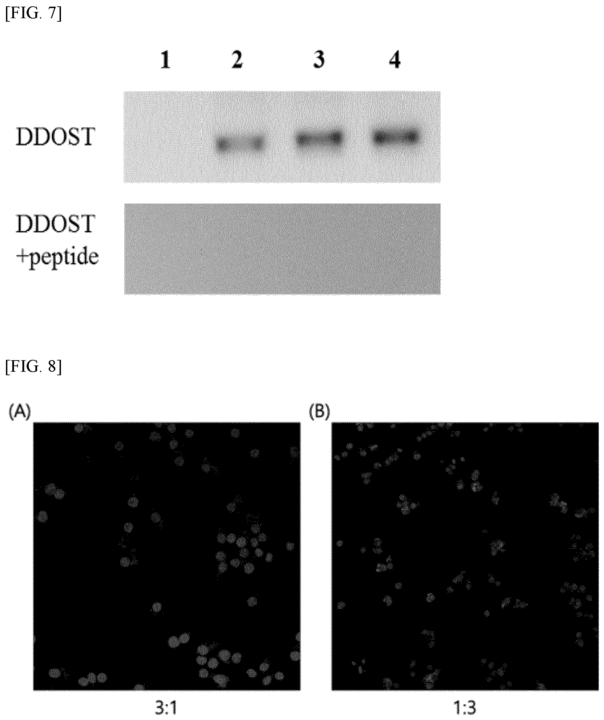

FIG. 7 is a set of images comparing nanoparticles according to the present invention and antibodies formed by the KLH conjugate method. To compare the production amount, as a result of comparing the case of inducing antibodies by combining with keyhole limpet hemocyanin, which is generally used to induce antibodies with soluble incomplete antigens, when only soluble incomplete antigens were injected, no antibodies were produced (lane 1 in the top panel of FIG. 7), whereas it was confirmed that antibodies were produced even when antibodies were induced by combining keyhole limpet hemocyanin (lane 2 in the top panel of FIG. 7). It was confirmed that when antibodies were induced using the nanoparticles of the present invention, a larger amount of antibodies could be induced compared to the previously widely used keyhole limpet hemocyanin method (lanes 3 and 4 of the upper panel of FIG. 7).
FIG. 8 is a set of images of nanoparticles internalized in macrophages photographed with a confocal fluorescence microscope. Macrophages were incubated with nanoparticles for 2 hours (concentration of nanoparticles: 40 µg/mL). Hapten was indicated by red fluorescence. Nucleus was counterstained in blue. (A) Amphiphilic peptide: Hapten peptide 3:1 (B) Amphiphilic peptide: Hapten peptide = 1:3.

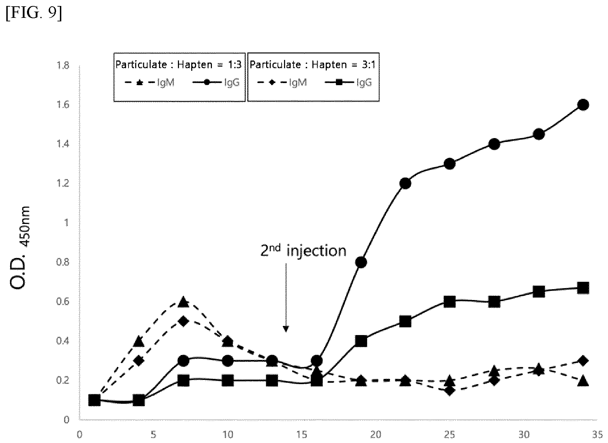

FIG. 9 is a titration curve for hapten peptides, and is a graph confirming specific IgG responses. BALB/c mice were immunized with 3:1(w/w) nanoparticles or 1:3(w/w) nanoparticles supplemented with carrier protein, hapten and adjuvant on days 0 and 21, twice a week. Blood was collected twice a week and analyzed by ELISA (in FIG. 9, particulates refer to amphiphilic peptides.)

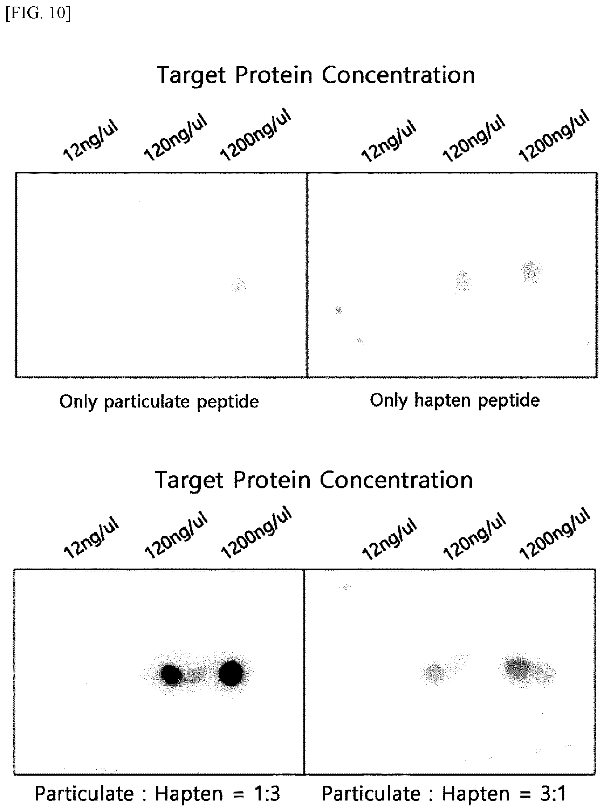

FIG. 10 is a diagram confirming the effect of nanoparticles on antigen-specific IgG response. BALB/c mice were immunized with emulsified 3:1 (w/w) or 1:3 (w/w) nanoparticles on days 0 and 21. On day 35, mice were sacrificed, and whole blood was collected. Serum was analyzed by dot blot for total protein, including haptens as a fraction (in FIG. 10, particulates refer to amphiphilic peptides).

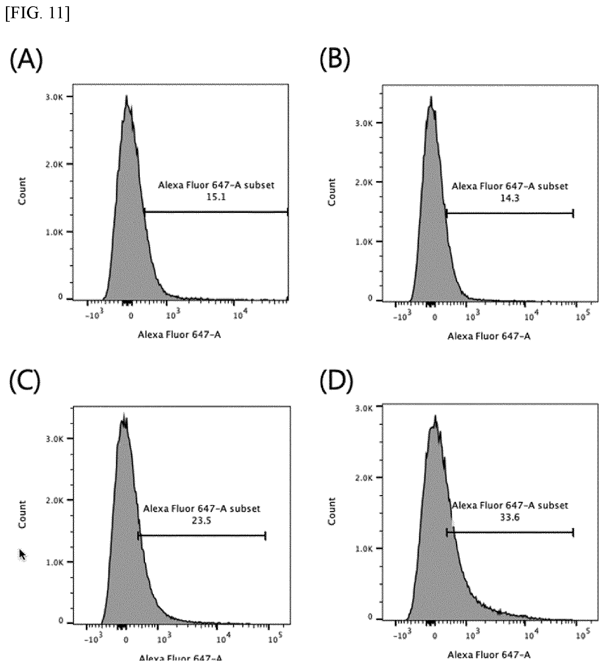

FIG. 11 is a histogram image of B cells reacting with DDOST protein labeled with Alexa 647 among splenocyte B cells according to an embodiment of the present invention. In this experiment, flow cytometric analysis of spleen cells harvested from immunized mice was performed. The Alexa 647 positive channel was used for the detection of hapten-specific B cells. In addition, hapten-specific B cells were the lower gate. A of FIG. 11 is the result for the amphiphilic peptide (Carrier), and the lower gating group was found to be 15.1%. B of FIG. 11 is the result for the hapten peptide, and the lower gating group was found to be 14.3%. C in FIG. 11 is the result for (amphiphilic peptide : hapten peptide) 3:1 (w/w) nanoparticles, and the lower gating group was found to be 33.6%. D of FIG. 11 is the result for 1:3 (w/w) nanoparticles, and the lower gating group was found to be 23.5%.

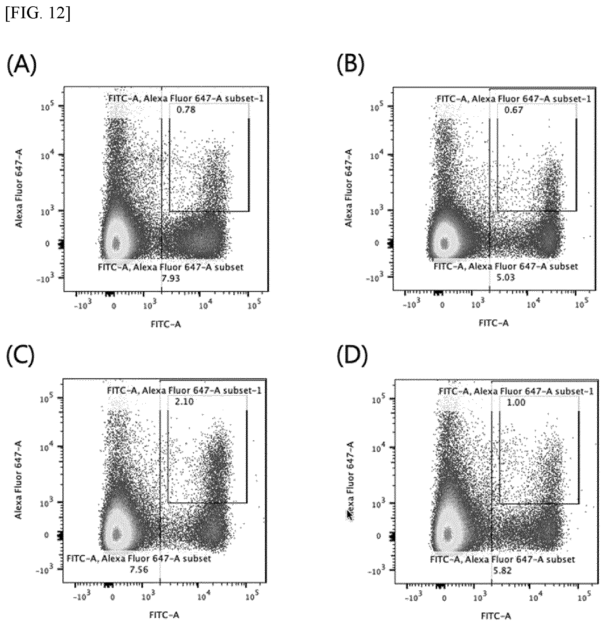

FIG. 12 shows the results of flow cytometric analysis of the hapten-specific cytotoxic T cell population. Splenocytes harvested from immunized mice were analyzed by flow cytometry. The Alexa 647 positive channel was used for the detection of hapten-specific cytotoxic T cells. In addition, hapten and CD8 specific T cells were the lower gates. A in FIG. 12 is the carrier analysis result, and the lower gating group showed 0.78%. B in FIG. 12 is the hapten analysis result, and the lower gating group showed 0.67%. C in FIG. 12 is the result of 3:1 (w/w) nanoparticle analysis, and the lower gating group showed 1%. D in FIG. 12 is the result of 1:3 (w/w) nanoparticle analysis, and the lower gating group showed 2.1%.

### Modes of the Invention

Hereinafter, the present invention will be described in more detail through examples. However, these examples are for illustrative purposes only, and the scope of the present invention is not limited to these examples and experimental examples.

### Example and Experimental Example

### Experimental Example 1. Materials and methods

### 1.1 Preparation of nanoparticles (particulate soluble haptens) using amphiphilic peptides

Recombinant amphiphilic peptides and hapten peptides were produced and provided by Anygen in Gwangju, Korea. In order to prepare nanoparticles (particulate soluble hapten) using amphiphilic peptides, amphiphilic peptides (peptides of SEQ ID NOs: 1 to 4, respectively) were dissolved in distilled water at a concentration of 4 mg/mL. Then, hapten peptides (peptides of SEQ ID NOs: 5 to 11, respectively) were dissolved in distilled water at a concentration of 0.5 mg/mL. Afterwards, a fixed amount of hapten (50 µg) was mixed with two different amounts of amphiphilic peptide at a ratio of 3:1 and 1:3 (w/w) and incubated for 30 minutes at room temperature (25°C).

### 1.2 Characterization of nanoparticle size

In order to characterize the sizes of amphiphilic peptides and nanoparticles, the diameters of micelles and nanoparticles formed by amphiphilic peptides (carriers) were measured with a Zetasizer Nano ZS system (Malvern Instruments, Malvern, UK). After digestion, the micelle stock solution was added to the cuvette. After 30-minute incubation, nanoparticles were diluted to a final volume of 1 mL. The average size was then analyzed with Dispersion Technology software 4.3. Nanoparticles were diluted using ultrapure water (refractive index 1.33, viscosity 0.89) and used for analysis at room temperature. Observations were presented as triplicate means +/standard deviation.

### 1.3 Cellular uptake of nanoparticles

Macrophages, RAW264.7, were purchased from ATCC, Virginia, USA. In order to detect nanoparticle uptake in cells, nanoparticles were labeled with Alexa 647. After labeling, the nanoparticles were incubated with a carrier, and the nanoparticles were prepared at 40 µg/mL. Cells were seeded in 8 chamber slides at 1 × 10⁴, and set for day 1. The cells were treated with two different ratios of nanoparticles at 1:3 or 3:1 for 2 hours. The cells were fixed at 4% CO for 10 minutes. The cells were blocked with 1% bovine serum albumin for 30 minutes. DAPI counterstaining was performed for 1 minute without antibody treatment. After mounting the cover glass, the cells were observed using a confocal fluorescence microscope SP5 TCS (Leica biosystems, USA).

### 1.4 Immunization of mice

Female BALB/C mice were purchased from Nara Biotech, Seoul, Korea, were 6 weeks old, and were bred under pathogen-free conditions. This study was conducted in accordance with the strict guidelines for the care and use of laboratory animals of the National Institutes of Health. The protocol was approved by the Animal Care Committee of Hanyang University Animal Hospital. For immunization, nanoparticles were emulsified with aluminum hydroxide (20 mg/100 µL, Sigma-Aldrich, St Louis, MO, USA). The mice were injected with 150 µg carrier dissolved in 150 µL phosphate buffered saline (PBS). The mice were injected with 50 µg hapten dissolved in 150 µL PBS. The mice were injected with 3:1 (w/w) nanoparticles (150 µg carrier and 50 µg hapten) combined with 150 µL PBS and 40 mg aluminum hydroxide. The mice were injected with 1:3 (w/w) nanoparticles (16.67 µg carrier and 50 µg hapten) combined with 150 µL PBS and 40 mg aluminum hydroxide. All groups were injected by intraperitoneal route and again on day 21. Blood was collected through the jugular vein twice a week and centrifuged at 2,000 g for 20 minutes. Serum was collected from centrifuged blood and frozen at -20°C until being analyzed by ELSIA. The mice were sacrificed on day 35. Whole blood was collected and centrifuged at 2,000 g for 20 minutes. Serum was collected from centrifuged blood and frozen at -20°C until being analyzed by dot blot. Spleens were collected to harvest spleen cells for flow cytometry.

### 1.6 Antibody detection by dot blot

For determination of nanoparticle (or hapten peptide)-specific antibodies, serial dilutions of the recombinant protein were seeded on PVDF membranes at a concentration of 1.2 µg/µL to 12 ng/µL. Membranes were blocked with 2.5% skim milk in PBS (blocking buffer) for 2 hours at room temperature. 20 µL serum was diluted with blocking buffer to a final volume of 3 mL and processed on the membrane for 2 hours at room temperature. After serum treatment, wells were incubated with horseradish peroxidase-conjugated anti-mouse IgG antibody for 1 hour at room temperature. Afterwards, the enhanced chemiluminescence solution (ECL) was treated with a membrane and directly analyzed with chemiscope 3400 (CLINX, China). Dots were visualized at 10 seconds. Membranes were washed three times with wash buffer (PBS with Tween) at all steps.

### 1.7 Spleen cell harvest

Spleens were collected from the mice sacrificed on day 35 and stored in RPMI 1640 (Coring, Kennebunk ME, USA) with 10% FBS without antibiotics. A 70 µm cell strainer was placed in a 50 mL conical tube and washed with 5 mL RPMI 1640. The spleen was cut out with scissors, placed in a cell strainer and gently triturated. The strainer was washed again with 5 mL RPMI 1640. After transferring the suspended cells to a 15 mL conical tube, the sample was stored on ice. Suspended cells were centrifuged at 550 g for 5 minutes, and the supernatant was discarded. 1 mL ACK lysis buffer (ThermoFisher Scientific, Massachusetts, USA) was treated in a 15 mL conical tube and incubated for 5 minutes at room temperature. 4 mL RPMI 1640 was added to the conical tube, and the suspended cells were centrifuged at 550 g for 5 minutes. This step was repeated until the red spots disappeared from the cell pellet. Finally, the cell pellet was resuspended in 5 mL RPMI 1640 and used directly for flow cytometry.

### 1.8 Live cell flow cytometry

In order to identify nanoparticle (or hapten peptide)-specific lymphocyte populations, live cell flow cytometry was performed. For the detection of hapten-specific B lymphocytes and T lymphocytes, DDOST recombinant protein including hapten peptides as a part was labeled with alexa 647 (ab269823, Abcam, Cambridge, UK). The collected spleen cells were centrifuged at 550 g for 5 minutes, and the supernatant was discarded. The cell pellet was resuspended in cold DPBS. This step was repeated three times for rinsing. After rinsing, the cells were filtered through a 40 µm cell strainer and counted. 2 × 10⁶ cells were transferred to a 1.75 mL E-tube. The cells were treated with FcR blocking reagent (for mice) for 10 minutes at 4°C.

In order to characterize B lymphocytes, alexa 488 conjugated anti-mouse cd19 antibody (ab270176, Abcam, Cambridge, UK) and alexa 647-labeled recombinant protein were treated with FcR blocking reagent (130-092-575, Miltenybiotec, Bergisch Gladbach, Germany) for 1 hour at 4°C. Treated cells were rinsed three times with FACS buffer. After rinsing, the cells were resuspended in 1 mL of cold DPBS. PI was added to the cells to detect viable cells for 10 minutes, and the cells were analyzed using FACS.

In order to characterize T lymphocytes, FITC-conjugated anti-mouse cd8 antibody (ab237367, Abcam, Cambridge, UK) and alexa 647-labeled recombinant protein were treated with FcR blocking reagent for 1 hour at 4°C. Treated cells were rinsed three times with FACS buffer. After rinsing, the cells were resuspended in 1 mL of cold DPBS. PI was added to the cells to detect viable cells for 10 minutes, and the cells were immediately analyzed by FACS conto II (BD biosciences, San Diego, USA). In all experiments for cell recovery, centrifugation was performed at 300 g for 3 minutes.

### Experimental Example 2. Results

### 2.1 Nanoparticle size measurement

First of all, in this example, the size of each nanoparticle formed by the amphiphilic peptide (R3V6) and the amphiphilic peptide (R3V6)-hapten peptide was measured and confirmed using an electron microscope (FIG. 4). Referring to FIG. 4, it can be confirmed that the amphiphilic peptide alone formed micelles (spherical particles) with a size between 50 and 300 nm (preferably, 100 and 300 nm), and when a hapten peptide was electrically attached to the micelle structure, it can be confirmed that particles having a size between 60 and 150 nm and 300 to 800 nm were formed depending on the ratio.

Additionally, in order to characterize the sizes of the carrier and nanoparticles, the diameters of micelles and nanoparticles formed by the amphiphilic peptide were measured. More specifically, a fixed amount of the hapten peptide was mixed with two different amounts of the amphiphilic peptide at the weight ratios of 3:1 and 1:3. The size of the micelles produced according to the present invention was 100 to 300 nm, the size of the 3:1 (w/w) nanoparticles was 60 to 150 nm, and the size of the 1:3 (w/w) nanoparticles was 300 to 800 nm (Table 1).

**[Table 1]**

| Weight ratio (Carrier:hapten) | Z-ave (d.nm) |
|---|---|
| Carrier | 193.8± 97.3 |
| 3:1 | 108.7± 48.2 |
| 1:3 | 562.5±247.4 |

### 2.2 Cellular uptake of nanoparticles

Since macrophages are one type of the antigen presenting cells, cellular uptake must occur when nanoparticles according to the present invention act as antigens.

In this example, the hapten peptide was labeled with the fluorescent substance alexa 647, mixed with the amphiphilic peptide (R3V6) in an adjusted ratio, and nanoparticles with average sizes of 30 to 50 nm and 200 to 300 nm, respectively, were treated with a macrophage cell line. Referring to FIG. 5, it can be confirmed that nanoparticles with a size of 200 to 300 nm easily entered macrophages, whereas the entry rate for nanoparticles with a size of 30 to 50 nm was significantly reduced. In other words, it can be seen that the effectiveness of the nanoparticles produced according to the present invention as a vaccine is improved when they are formed in a size of 150 to 350 nm.

In addition, the hapten peptide was mixed with two different amphiphilic peptides at a weight ratio of 3:1 (left image of FIG. 8) and 1:3 (right image of FIG. 8), and then each produced nanoparticle was treated with a macrophage cell line. After 2 hours of incubation, it was confirmed that the nanoparticles were absorbed into macrophages (FIG. 8).

### 2.3 Detection of nanoparticle (or hapten peptide)-specific antibodies

### 2.3.1 Increase in nanoparticle (or hapten peptide)-specific IgG antibodies

Blood was collected from mice twice a week, and ELISA was performed to detect an increase in nanoparticle (or hapten peptide)-specific IgG antibodies. IgM increased only in the group injected with a mixture of amphiphilic peptide (micellar structure) and hapten peptide, reaching the highest level for about 7 days and then rapidly decreasing. Although it is not shown in the drawings because it is complicated, there was no significant increase in antibody expression for any IgM IgG in the group injected only with the amphiphilic peptide or hapten peptide.

Specifically, nanoparticles with a size of 200 to 300 nm (A, ● in FIG. 6) and nanoparticles with a size of 30 to 50 nm (◆, ■ in FIG. 6) produced according to the present invention were injected into mice, and 15 days later, after the same amount was injected into the mice again, the expressions of target peptide (hapten peptide)-specific IgG and IgM antibodies in the mouse blood were confirmed (FIG. 6). Referring to FIG. 6, it can be confirmed that IgM and IgG specific to the hapten peptide were induced in the same manner as a typical vaccine.

In addition, the results of confirming the expressions of IgG and IgM antibodies according to the mixing ratio of amphiphilic peptide and hapten peptide are shown in FIG. 9. In the case of IgG, there was no significant increase after the first vaccination, but after the second booster injection, it was confirmed that the optical density (O.D.) increased in the group mixed with amphiphilic peptide and hapten peptide at a ratio of 1:3 (w/w). Meanwhile, in the group treated with a mixture of amphiphilic peptide and hapten peptide at a 3:1 (w/w) ratio, the increase in O.D was significantly lower than that of the 1:3 group (FIG. 9).

### 2.3.2 Comparison of modular vaccines and antibodies formed by the KLH conjugate method

When dot blot analysis was performed on the initially targeted protein using antiserum isolated from blood 33 days after vaccine injection, it was possible to confirm good results when 200 to 300 nm nanoparticles were administered, similar to the data from ELISA.

Meanwhile, in order to compare the amount of antibody production, as a result of comparing the case of inducing antibodies by combining with keyhole limpet hemocyanin, which is most commonly used when inducing antibodies with soluble incomplete antigens, it was confirmed that when only the hapten peptide was injected, no antibodies were produced (lane 1 in the top panel of FIG. 7), whereas it was confirmed that antibodies were produced when the antibody was induced by binding to keyhole limpet hemocyanin (lane 2 in the top panel of FIG. 7). It was confirmed that when antibodies were induced using the nanoparticles of the present invention, the same or even greater amount of antibodies could be induced compared to the previously widely used keyhole limpet hemocyanin method (lanes 3 and 4 in the top panel of FIG. 7).

In addition, when the antibodies identified by Western blot were pre-reacted with the incomplete water-soluble antigen (lower panel of FIG. 7), all bands disappeared, confirming that all antibodies that were produced were specifically generated against the incomplete water-soluble antigen. After completion of the experiment, the spleens of the used animals were removed, splenocytes were isolated, and the analysis of B cells and T cells was performed through flow cytometry.

### 2.3.3 Detection of nanoparticle-specific IgG antibodies

In order to identify nanoparticle (or hapten peptide)-specific IgG antibodies, whole blood was collected from mice sacrificed on day 35, and dot blot assay was performed. The dots of 1:3 (w/w) nanoparticles were confirmed to be the most obvious dots as shown in the previous ELISA experiment results, and it was confirmed that they were highly concentrated and diluted as the seeded recombinant protein concentration was diluted. However, no concentrated dots were found in the group in which only the amphiphilic peptide micelle structure or hapten peptide was separately injected. Meanwhile, in the group where the amphiphilic peptide and hapten were mixed at a 3:1 (w/w) ratio, a very light but distinct dot was confirmed (FIG. 10). As shown in the results of the previous ELISA experiment, it was confirmed that the group in which the amphiphilic peptide and hapten were mixed at a 3:1 (w/w) ratio had a lower antibody production rate than the group in which the amphiphilic peptide and hapten were mixed at a 1:3 (w/w) ratio.

### 2.4 Identification of nanoparticle-specific lymphocyte populations

### 2.4.1 Nanoparticle-specific B cell population

In order to identify nanoparticle (or hapten peptide)-specific B cell populations, live cell flow cytometric analysis was performed using a recombinant protein labeled with alexa 647, which includes haptens as part. After gating on CD19 positive B cells, the histogram was set to the alexa 647 positive channel and sub-gated with bars (FIG. 11).

The group treated with the amphiphilic peptide alone (FIG. 11, A) and the group treated with the hapten peptide alone (FIG. 11, B) showed no significant change in the ratio of cells responding to the target protein, but in the group where the amphiphilic peptide and hapten peptide were mixed at a 3:1 ratio (FIG. 11, C), the proportion of cells responding to the target protein was 8% higher than in the group treated with the amphiphilic peptide or hapten peptide. Additionally, in the group where the amphiphilic peptide and hapten peptide were mixed at a 1:3 ratio (FIG. 11, D), the proportion of cells responding to the target protein was approximately 18% greater than the group treated with the amphiphilic peptide or hapten peptide (see Table 2).

**[Table 2]**

| B cell | 1:3(w/w) particulate hapten | 3:1(w/w) particulate hapten |
|---|---|---|
| % point | 13.15±5.35 | 9.15±0.75 |

### 2.4.2 Hapten-specific cytotoxic T cell population

In order to identify hapten-specific T cell populations, an alexa 647-labeled recombinant protein including hapten as a part was used, and live cell flow cytometry was performed. A dot plot is displayed, and each axis represents a target specific channel (X axis - CD8 specific channel, Y axis - hapten specific channel). Similar to the B cell analysis, the group treated with the amphiphilic peptide alone (FIG. 12, A) and the group treated with the hapten peptide alone (FIG. 12, B) did not show significant changes in the proportion of cells reacting to the target protein, but in the amphiphilic peptide: hapten peptide = 1:3 group, it was 0.22% higher than the amphiphilic peptide alone injection and 0.33% higher than the hapten peptide only injection group (FIG. 12 A, B and D). In comparison, in the amphiphilic peptide:hapten peptide = 3:1 group, it was 1.32% higher than the group injected with the amphiphilic peptide alone and 1.43% higher than the group injected with the hapten peptide alone (FIG. 12 A, B and D), and thus, it was confirmed that the group with a relatively small particle size can easily increase cytotoxic T cells.

Hereinabove, preferred embodiments for the present invention have been described. One of ordinary skill in the art to which the present invention pertains will understand that the present invention may be implemented in a modified form without departing from the essential features of the present invention. Therefore, the disclosed embodiments should be considered in descriptive sense only and not for purposes of limitation. The scope of the present invention is set forth in the claims rather than the foregoing description, and all differences within the scope equivalent thereto should be construed as being included in the present invention.

### Sequence Listing Free Text

SEQ ID NO: 1
   amphiphilic peptide R3V6
   RRRVVVVVV
SEQ ID NO: 2
   amphiphilic peptide R4V7
   RRRRVVVVVVV
SEQ ID NO: 3
   amphiphilic peptide E3V6
   EEEVVVVVV
SEQ ID NO: 4
   amphiphilic peptide E4V7
   EEEEVVVVVVV
SEQ ID NO: 5
   hapten peptide 1
   SDLGQHTLIV
SEQ ID NO: 6
   hapten peptide 2
   LEDTLSSE
SEQ ID NO: 7
   hapten peptide 3
   PGSQRYSQTGN
SEQ ID NO: 8
   Dengue virus subtype1 hapten peptide
   APTSEIQLTD
SEQ ID NO: 9
   Dengue virus subtype2 hapten peptide
   SSITEAELTG
SEQ ID NO: 10
   Dengue virus subtype3 hapten peptide
   ASTVEAILPE
SEQ ID NO: 11
   Dengue virus subtype4 hapten peptide
   SPSVEVKLPD

## Claims

1. A nanoparticle, comprising an amphiphilic peptide that forms a micelle structure through self-assembly, and a target peptide that electrically binds to a surface of the amphiphilic peptide.

2. The nanoparticle of claim 1, wherein the amphiphilic peptide is a peptide represented by General Formula I below:
[General Formula I] CnXm
wherein C is a charged amino acid, X is a hydrophobic amino acid, n is an integer from 1 to 5, and m is an integer from 3 to 10.

3. The nanoparticle of claim 1, wherein the amphiphilic peptide comprises arginine (R) as a hydrophilic amino acid and valine (V) as a hydrophobic amino acid.

4. The nanoparticle of claim 1, wherein the amphiphilic peptide is at least one selected from the group consisting of peptides represented by amino acid sequences of SEQ ID NO: 1 to SEQ ID NO: 4.

5. The nanoparticle of claim 1, wherein the target peptide has a polarity opposite to a surface polarity of the micelle structure.

6. The nanoparticle of claim 1, wherein the target peptide is an antigen peptide.

7. The nanoparticle of claim 6, wherein the antigen peptide is a hapten peptide of a target antigen.

8. The nanoparticle of claim 1, wherein the target peptide consists of any one of amino acid sequences of SEQ ID NO: 5 to SEQ ID NO: 11.

9. The nanoparticle of claim 1, wherein the amphiphilic peptide and the target peptide are comprised at a weight ratio of 1:5 to 5:1.

10. The nanoparticle of claim 1, wherein the size of the nanoparticle is 50 nm to 1,000 nm.

11. A vaccine composition, comprising the nanoparticle of claim 1.

12. The vaccine composition of claim 11, wherein the vaccine composition is for preventing dengue virus infection, severe fever with thrombocytopenia syndrome or influenza virus infection.

13. A method for preparing a nanoparticle, comprising:
a) forming a micelle structure by mixing an amphiphilic peptide in an aqueous solution; and
b) mixing a target peptide to electrically bind to a surface of the formed micelle structure.

14. The method of claim 13, wherein the amphiphilic peptide is a peptide represented by General Formula I below:
[General Formula I] CnXm
wherein C is a charged amino acid, X is a hydrophobic amino acid, n is an integer from 1 to 5, and m is an integer from 3 to 10.

15. The method of claim 13, wherein the amphiphilic peptide and the target peptide of step b) are mixed at a weight ratio of 1:5 to 5:1.

16. The method of claim 13, wherein the target peptide is a hapten peptide.
